(19) Europäisches Patentamt
European Patent Office
Office européen des brevets

(11) **EP 0 525 960 B1**

(12) **EUROPEAN PATENT SPECIFICATION**

(45) Date of publication and mention
of the grant of the patent:
**20.03.1996 Bulletin 1996/12**

(51) Int. Cl.$^6$: **A61K 35/74**

(21) Application number: **92305287.2**

(22) Date of filing: **09.06.1992**

(54) **Use of rapamycin for the treatment of adult T-cell leukemia/lymphoma**

Verwendung von Rapamycin zur Behandlung von T-Zellen Lymphom/Leukämie bei Erwachsenen

Utilisation de rapamycine pour le traitement de la lymphome/leucéme des cellules T adultes

(84) Designated Contracting States:
**AT BE CH DE DK ES FR GB GR IT LI LU NL PT SE**

(30) Priority: **18.06.1991 US 717773**

(43) Date of publication of application:
**03.02.1993 Bulletin 1993/05**

(73) Proprietor: **AMERICAN HOME PRODUCTS CORPORATION**
**Madison, New Jersey 07940-0874 (US)**

(72) Inventors:
• **Miller, Glenn Alvin**
**Miami, FL (US)**
• **Rabin, Mark Barry**
**Miami, FL (US)**
• **Harrington, William Joseph, Jnr.**
**Miami, FL (US)**

(74) Representative: **Wileman, David Francis, Dr. et al**
**c/o Wyeth Laboratories**
**Huntercombe Lane South**
**Taplow Maidenhead Berkshire SL6 OPH (GB)**

(56) References cited:
BE-A- 877 700

• JOURNAL OF IMMUNOLOGY, vol. 148, no. 10, 15 May 1992, American Association of Immunologists, US; P. HÖLLSBERG et al., pp. 3256-3263
• JOURNAL OF IMMUNOLOGY, vol. 144, no. 4, 15 February 1990, American Association of Immunologists, US; F.J. DUMONT et al., pp. 1418-1424
• TRANSPLANTATION PROCEEDINGS, vol. 23, no. 2 (suppl. 2), 1991, Proceedings of a Satellite Symposium on Immunosuppressive Drugs, Targets and Mechanisms of Action, San Francisco, CA (US), 19 August 1990; N.H. SIGAL et al., pp. 1-5
• JOURNAL OF NEUROIMMUNOLOGY, vol. 0, suppl. 1, 1991, 3rd Internatl. Conference on Neuroimmunology, Jerusalem (IL), 27 October-01 November 1991; P. HÖLLSBERG et al., p. 201
• TRENDS IN PHARMACOLOGICAL SCIENCES, vol. 12, no. 6, June 1991, Elsevier Science Publishers Ltd., GB; J.Y. CHANG et al., pp. 218-223

## Description

This invention relates to a method of treating adult T-cell leukemia/lymphoma, in particular to a new therapeutic use of rapamycin.

Adult T-cell Leukemia/Lymphoma (ATL) is an aggressive hematologic disease, characterized by a rapid, invariably fatal course. Clinical features of ATL include hypercalcemia, bone marrow infiltration, generalized lymphadenopathy, leukemia, skin involvement, hepatosplenomegaly and lytic bone lesions.

The human T-lymphotropic virus 1 (HTLV-1) is the etiologic agent of ATL. Evidence linking HTLV-1 to ATL includes epidemiologic clustering of ATL in geographic regions endemic for HTLV-1 infection [Hinuma, Y., Proc. Natl. Acad. Sci. 78: 6476 (1981); Watanabe, T., Virology 133: 238 (1984); Catovsky, D. Lancet 1: 639 (1982); Blattner, W.A., Int. J. Ca. 30: 257 (1982)], the high frequency of specific antibodies in sera from ATL patients, [Hinuma, Y., Proc. Natl. Acad. Sci. 78: 6476 (1981)], detection of monoclonally integrated proviral sequences in leukemic cells of patients with this disease [Yoshida M., Proc. Natl. Acad. Sci. 81: 2534 (1984)], and isolation of infectious virus from leukemia cell cultures [Poiesz, B.J., Proc. Natl. Acad. Sci. 77: 7415 (1980); Yoshida, M., Proc. Natl. Acad. Sci. 79: 2031 (1982)].

HTLV-1 transformed T-cells are characterized by uncontrolled proliferation in the absence of antigenic stimulation. IL-2 independent growth is a hallmark of these cells in culture. The cell surface phenotype of these cells is dominated by a significant increase in IL-2 receptors. Recent evidence demonstrated that these receptors are high affinity IL-2R. The majority of T-cells isolated from infected individuals tested did not, however, respond to exogenously added IL-2. [Kodaka, T., Jpn. J. Ca. Res. 81: 902 (1990)].

HTLV-1 has been reported to be transmitted sexually, from mother-child via breast feeding, among intravenous drug abusers (presumably by sharing of contaminated needles), and via blood transfusion [Hino, S., Gann 76: 474 (1985); Murphy, E.L., Ann. Int. Med. 111: 555 (1989); Sato, H., Int. J. Ca. 37: 395 (1986); Robert-Guroff, M., J.A.M.A., 255: 3133 (1986)]. Infection with the virus has an associated lifetime risk for developing ATL estimated to be approximately 4% [Murphy, E.L., Ann. Int. Med. 111: 555 (1989)].

The prevalence of HTLV-1 infection in residents of the United States has recently become a public health concern. An epidemiologic survey undertaken in major cities of the United States detected average seroprevalence rates of 0.025 percent among healthy blood donors [Williams, A.E., Science, 240: 643 (1988)]. More recent epidemiologic data indicate a seropositivity rate of 4.4 percent among healthy blacks in Brooklyn, New York suggesting seroprevalence may vary significantly depending on the population sampled. [Dosik, H., Proc. Am. Soc. Clin. Oncol. 9: 2 (1990)]. Khabbaz [J.A.M.A. 263: 60 (1990)] reported average seroprevalence rates of 6.7% among prostitutes nationwide. These data ranged from a low of 0% in Nevada to a high of 25.4% in Newark, NJ indicating variable rates even among high risk groups.

Internationally, HTLV-1 is present in endemic proportions in several areas of the world. The southwestern islands of Japan have long been identified as an endemic region for HTLV-1 infection. [Jpn. J. Clin. Oncol. 15: 517 (1985)]. For example, seroprevalence rates as high as 8% have been detected in healthy volunteer blood donors in the southern providence of Kyushu. [Maeda, Y., Int. J. Ca. 33: 717 (1984)]. A seroprevalence rate of 9.2% among otherwise healthy female food service workers over the age of 30 was reported in Jamaica. [Murphy, E.L., Ann. Int. Med. 111: 555 (1989)]. The seroprevalence rate rose to 14.1% in age matched females seen at a Jamaican sexually transmitted disease clinic. Risk factors for infection with the virus follow the expected pattern of a sexually transmitted disease.

Although serological and molecular genetic tests are available to screen for HTLV-1 infection, there is currently no means of early diagnosis of ATL. Presently, there is no effective therapeutic treatment for late stage disease, and as such mean survival after diagnosis is less than 6 months. Unsuccessful treatments were reported using radiation; methotrexate; leukovorin; interferon SQ; high dose steroids; and combinations containing cyclophosphamide, adriamycin, oncovin, prednisone, and bleomycin therapies. [Harrington, J. Aids 4: 284 (1991)]. Efficacious treatment of ATL was reported with SUN4800 (recombinant interferon gamma). [Ishihara, K., J. Invest. Dermatol. 93: 556 (1989)]. Responses of 90% were reported in 3 of 10 ATL patients treated with anti-Tac, a mouse antibody against the human p55 IL-2 receptor peptide (Tac; CD25). [Junghans, R., 37: 861A (1989)].

Rapamycin, a macrocyclic triene antibiotic produced by Streptomyces hygroscopicus [U.S. Patent 3,929,992] has been shown to prevent the formation of humoral (IgE-like) antibodies in response to an albumin allergic challenge [Martel, R., Can. J. Physiol. Pharm. 55: 48 (1977)], inhibit murine T-cell activation [Strauch, M., FASEB 3: 3411 (1989)], and prolong survival time of organ grafts in histoincompatible rodents [Morris, R., Med. Sci. Res. 17: 877 (1989)].

This invention provides a method of treating adult T-cell leukemia/lymphoma (ATL) in a mammal in need thereof which comprises administering an antiproliferative amount of rapamycin orally, parenterally, intranasally, intrabronchially, topically, transdermally, or rectally. In particular, rapamycin is useful in arresting the progression of and inducing remission of ATL by virtue of its ability to inhibit the proliferation of HTLV-1 transformed T-cells.

This invention also provides use of rapamycin in the preparation of a medicament for treating adult T-cell leukemia/lymphoma in a mammal.

The effect of rapamycin on ATL was established in an in vitro standard pharmacological test procedure using ten human HTLV-1 transformed T-cell lines cultured from ATL patients. These cell lines are presently the best models for ATL in humans. Cyclosporin A (CsA) was also evaluated for the purpose of comparison. The procedure used and results obtained are described below.

ATL cell lines were established according to known literature procedures. ATL5S, ATL3I, and ATL1K were established according to the method of Hoshino [Proc. Natl. Acad. Sci, 80: 6061(1983)]. The cell lines 31008, 31009, 31016 and AT(EGG) were established from peripheral blood of HTLV-1 infected individuals via co-culture with cord blood lymphocytes. The cell line MALN was isolated from lymphoid tumor tissue of an HTLV-1 infected ATL patient seen at Jackson Memorial/University of Miami Medical Center. [Harrington, W.J., Jr., J. Acq. Imm. Def. Dis., 4: 284 (1991)]. The primary cultures BL and GP were initiated from peripheral blood of ATL patients. The MALN, BL, and GP cell lines were initiated without co-cultivation.

Cells were adjusted to a concentration of $1 \times 10^6$ cells/mL in RPMI 1640 with 15% fetal bovine serum and were supplemented with 50 U/mL penicillin, 50 μg/mL streptomycin, and 2 mM glutamine. Rapamycin and CsA were each diluted to a stock concentration of 1 mM in absolute ethanol with subsequent dilutions being made in culture medium. Drug concentrations evaluated ranged from 1μM to 100 fM. Cultures were incubated at 37°C, 5% $CO_2$ for 24 hours followed by a 4 hr. pulse with 0.5 μCi of tritiated thymidine. Thymidine incorporation was measured by liquid scintillation counting, and the results expressed in counts per minute (cpm). The results obtained from the mean of four evaluations at each concentration were compared with control values to determine percent inhibition. The following formula was used to calculate the percent inhibition:

$$\text{Percent Inhibition} = \frac{\text{cpm[no drug]} - \text{cpm[drug]}}{\text{cpm[no drug]}} \times 100$$

The percent inhibition obtained with rapamycin is shown in the following table.

| PERCENT INHIBITION OF ATL CELL PROLIFERATION | | | | | | | | | | |
|---|---|---|---|---|---|---|---|---|---|---|
| Rapamycin Concentration | Cell Line | | | | | | | | | |
| | ATL3I | ATL5S | ATL1K | ATEGG | 31008 | 31009 | 31016 | MALN | GP | BL |
| 1 μM | 28 | 59 | 19 | 58 | 69 | 71 | 59 | 55 | 54 | 80 |
| 100 nM | 17 | 58 | 32 | 60 | 68 | 70 | 56 | 54 | 46 | 79 |
| 10 nM | 15 | 49 | 18 | 45 | 58 | 67 | 49 | 41 | 46 | 76 |
| 1 nM | 18 | 48 | 26 | 53 | 54 | 68 | 48 | 43 | 42 | 73 |
| 100 pM | 11 | 47 | 23 | 49 | 45 | 61 | 49 | 37 | 39 | 65 |
| 10 pM | 6 | 38 | 12 | 44 | 31 | 47 | 47 | 26 | 14 | 55 |
| 1 pM | 11 | 14 | 15 | 48 | 30 | 59 | 50 | 36 | 20 | 56 |
| 100 fM | 7 | 36 | 15 | 44 | 29 | 60 | 44 | 36 | 23 | 51 |

Rapamycin inhibited proliferation of 8 of the 10 ATL cell lines at a concentration of 100 nM or greater, with a 50% reduction in proliferation in 4 of the cell lines at a concentration of 1 nM. Both of the cell lines in which proliferation was not inhibited by rapamycin had been in culture for more than 5 years without recloning; their nonresponsiveness to rapamycin may be attributed to their further transformation into insensitive clones during this time period. In comparison, CsA did not inhibit proliferation in 7 of the 10 cell lines in which it was evaluated. In the remaining 3 cell lines (ATL3I, ATL5S, and 31009) proliferation was inhibited by 16%, 23%, and 22%, respectively.

The results obtained in this standard pharmacological test procedure demonstrate that rapamycin inhibits the proliferation of HTLV-1 transformed T-cell lines, which are presently the best models for ATL in humans, and as such, rapamycin is useful in the treatment of ATL.

When rapamycin is employed in the treatment of ATL, it can be formulated neat or with a pharmaceutical carrier to a mammal in need thereof preferably in unit dosage form; conveniently a single unit dose for daily administration. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellent.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

Rapamycin may be administered rectally in the form of a conventional suppository. For administration by intranasal or intrabronchial inhalation or insufflation, rapamycin may be formulated into an aqueous or partially aqueous solution, which can then be utilized in the form of an aerosol. Rapamycin may also be administered transdermally through the use of a transdermal patch containing the active compound and a carrier that is inert to the active compound, is non-toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

Rapamycin may be administered topically as a solution, cream, or lotion by formulation with pharmaceutically acceptable vehicles containing 0.1 - 5 percent, preferably 2%, of active compound.

The dosage requirements vary with the particular compositions employed, the route of administration, the severity of the symptoms presented and the particular subject being treated. Based on the results obtained in the standard pharmacological test procedure, projected parenteral daily dosages of active compound would be 0.01 μg/kg - 10 mg/kg and preferably between 0.1 μg/kg - 2 mg/kg. When rapamycin is given parenterally, it will preferably be administered intravenously. Projected oral daily dosages of active compound would be 1 μg/kg - 15 mg/kg and preferably between 0.01 mg/kg - 10 mg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. In general, rapamycin is most desirably administered at a concentration that will generally afford effective results without causing any harmful or deleterious side effects, and can be administered either as a single unit dose, or if desired, the dosage may be divided into convenient subunits administered at suitable times throughout the day.

For the treatment of ATL, it is also contemplated that rapamycin may be administered in conjunction with chemotherapeutic agents such as methotrexate, leukovorin, cyclophosphamide, adriamycin, oncovin, prednisone, bleomycin, and the like; high dose steroids; radiation therapy; cytokine therapy, such as interferon; monoclonal antibody therapy; and other therapies useful in the treatment of neoplastic diseases.

## Claims

1. Use of rapamycin in the preparation of a medicament for treating adult T-cell leukemia/lymphoma in a mammal.

2. Use of rapamycin according to Claim 1 in which the medicament is adapted for administration orally, parenterally, intranasally, intrabronchially, topically, transdermally or rectally.

3. Use of rapamycin according to Claim 2 in which the medicament is adapted for intravenous administration.

4. Use of rapamycin according to Claim 3 in which the medicament is in unit dosage form to provide a daily dosage from 0.01 μg/kg to 10 mg/kg based on the weight of the mammal to treated.

**5.** Use of rapamycin according to Claim 3 in which the medicament is in unit dosage form to provide a daily dosage from 0.1 µg/kg to 2 mg/kg based on the weight of the mammal to treated.

**6.** Use of rapamycin according to Claim 1 in which the medicament is adapted for oral administration.

**7.** Use of rapamycin according to Claim 6 in which the medicament is in unit dosage form to provide a daily dosage from 1 µg/kg to 15 mg/kg based on the weight of the mammal to treated.

**8.** Use of rapamycin according to Claim 6 in which the medicament is in unit dosage form to provide a daily dosage from 0.01 mg/kg to 10 mg/kg based on the weight of the mammal to treated.

**Patentansprüche**

**1.** Verwendung von Rapamycin bei der Herstellung eines Medikaments zur Behandlung von adulter T-Zell-Leukämie/Lymphom bei Säugern.

**2.** Verwendung von Rapamycin nach Anspruch 1, wobei das Medikamet zur oralen, parenteralen, intranasalen, intrabronchialen, topischen, transdermalen oder rektalen Verabreichung geeignet ist.

**3.** Verwendung von Rapamycin nach Anspruch 2, wobei das Medikament zur intravenösen Verabreichung geeignet ist.

**4.** Verwendung von Rapamycin nach Anspruch 3, wobei das Medikament in Einheitsdosierungsform vorliegt, um eine tägliche Dosierung von 0,01 µg/kg bis 10 mg/kg, bezogen auf das Gewicht des zu behandelnden Säugers, vorzusehen.

**5.** Verwendung von Rapamycin nach Anspruch 3, wobei das Medikament in Einheitsdosierungsform vorliegt, um eine tägliche Dosierung von 0,1 µg/kg bis 2 mg/kg, bezogen auf das Gewicht des zu behandelnden Säugers, vorzusehen.

**6.** Verwendung von Rapamycin nach Anspruch 1, wobei das Medikamet zur oralen Verabreichung geeignet ist.

**7.** Verwendung von Rapamycin nach Anspruch 6, wobei das Medikament in Einheitsdosierungsform vorliegt, um eine tägliche Dosierung von 1 µg/kg bis 15 mg/kg, bezogen auf das Gewicht des zu behandelnden Säugers, vorzusehen.

**8.** Verwendung von Rapamycin nach Anspruch 6, wobei das Medikament in Einheitsdosierungsform vorliegt, um eine tägliche Dosierung von 0,01 mg/kg bis 10 mg/kg, bezogen auf das Gewicht des zu behandelnden Säugers, vorzusehen.

**Revendications**

**1.** Utilisation de la rapamycine dans la préparation d'un médicament pour le traitement de la lymphome/leucémie des cellules T adultes chez un mammifère.

**2.** Utilisation de la rapamycine suivant la revendication 1, dans laquelle le médicament est adapté pour l'administration par voie orale, parentérale, intranasale, intrabronchique, topique, transdermique ou rectale.

**3.** Utilisation de la rapamycine suivant la revendication 2, dans laquelle le médicament est adapté pour l'administration par voie intraveineuse.

**4.** Utilisation de la rapamycine suivant la revendication 3, dans laquelle le médicament est sous forme de dosage unitaire pour fournir un dosage quotidien de 0,01 µg par kg à 10 mg par kg par rapport au poids du mammifère à traiter.

**5.** Utilisation de la rapamycine suivant la revendication 3, dans laquelle le médicament est sous forme de dosage unitaire pour fournir un dosage quotidien de 0,1 µg par kg à 2 mg par kg par rapport au poids du mammifère à traiter.

**6.** Utilisation de la rapamycine suivant la revendication 1, dans laquelle le médicament est adapté pour l'administration par voie orale.

7.  Utilisation de la rapamycine suivant la revendication 6, dans laquelle le médicament est sous forme de dosage unitaire pour fournir un dosage quotidien de 1 µg par kg à 15 mg par kg par rapport au poids du mammifère à traiter.

8.  Utilisation de la rapamycine suivant la revendication 6, dans laquelle le médicament est sous forme de dosage unitaire pour fournir un dosage quotidien de 0,01 mg par kg à 10 mg par kg par rapport au poids du mammifère à traiter.